# EUROPEAN PATENT SPECIFICATION

(11) **EP 3 383 834 B1**
(45) Date of publication and mention of the grant of the patent: **11.11.2020**
(21) Application number: 16819759.8
(22) Date of filing: 02.12.2016
(51) Int. Cl.: C07C 67/62, C07C 45/86, C07C 253/32, C07C 51/50, C07C 255/08, C07C 57/04, C07C 69/15, C07C 69/54, C07C 47/22

(54) **ADDITIVES FOR MITIGATING FOULING DURING THE OXIDATION OF ALKENES**
ADDITIVE ZUR VERRINGERUNG VON VERSCHMUTZUNG WÄHREND DER OXIDATION VON ALKENEN
ADDITIFS POUR L'ATÉNUATION DE L'ENCRASSEMENT LORS DE L'OXYDATION D'ALKENES

(30) Priority: 04.12.2015 US 201562263419 P; 01.12.2016 US 201615366758
(43) Date of publication of application: 10.10.2018
(73) Proprietor: Baker Hughes Holdings LLC, Houston, TX 77073 (US)
(72) Inventor: MO, Hua, Friendswood, TX 77546 (US); METZLER, Roger, D., Sugar Land, TX 77498 (US); HUANG, Hsi-Yi, Kaohsiung 80461 (TW)
(74) Representative: BRP Renaud & Partner mbB Rechtsanwälte Patentanwälte Steuerberater
(86) International application number: PCT/US2016/064698
(87) International publication number: WO 2017/096210

(56) References cited:
- EP-A1- 0 576 010
- EP-A1- 0 791 573

## Description

### Field of Invention

The present invention relates to mitigating, reducing or inhibiting fouling during the oxidation of alkenes, and more specifically relates to introducing an effective amount of an additive to mitigate, reduce, or inhibit fouling during the oxidation of alkenes.

### Background

Industrial methods for oxidizing alkenes to produce acrylates, including acrylonitrile, acrylic acid, vinylacetate, acrylates and acrolein, include separation and purification processes such as distillation to remove unwanted impurities or byproducts. Certain impurities, such as peroxides can undergo thermolysis and produce free radicals. These free radicals, in turn, catalyse unwanted polymerization of the alkenes, resulting in fouling caused by the buildup of polymer deposits.

EP 0 791 573 A1 refers to a method of inhibiting the polymerization of vinyl acetate in vinyl acetate production units, the method comprising adding from about 0.001 to about 5,000 parts per million of a nitroxide compound to a vinyl acetate production unit whereby the polymerization of vinyl acetate in the unit is inhibited and excess acetaldehyde is not produced.

EP 056 010 A1 refers to a method for reducing the fouling of a compressor of a vinyl acetate production unit having a light end stream feed, wherein a hindered phenol is added to the compressor feed. The method retards the loss of compressor efficiency with run time and increases the run time before clean-out is required. The hindered phenol is added in a dosage amount effective for reducing the fouling of the compressor.

Substituted phenol derivatives are potentially useful compounds to mitigate, reduce, or inhibit fouling.

### Summary

In one embodiment, the present invention provides a method, comprising: a. adding an effective amount of at least one compound selected from a substituted catechol to a solution of a monomer selected from the group consisting of acrylonitrile, acrylic acid, vinylacetate, acrylates and acrolein; b. placing the monomer solution containing the at least one compound in a pressure vessel, pressurizing the pressure vessel and removing oxygen from the pressure vessel, wherein the pressure within the pressure vessel is from -0.0965 MPa (-14 psi) to 9.652 MPa (1400 psi); and c. heating the monomer solution containing the at least one compound to a temperature from 50 °C to 140 °C, wherein the at least one compound inhibits fouling by preventing polymerization of the monomer.

The substituted catechol is a compound of Formula 1: 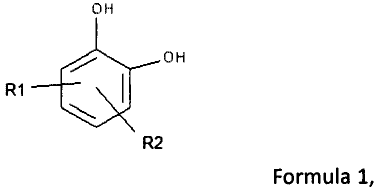 wherein R1 and R2 are independently selected from an alkyl group, an aryl group, an alkyl group, an alkyl group having a heteroatom, an aryl group having a heteroatom, and combinations thereof.

In one embodiment, the compound of Formula 1 is selected from the group consisting of 4-tert-Butylcatechol, and 3,5-tert-Butyl catechol.

In one embodiment, the monomer solution containing the at least one compound is heated for a period of time from 0.5 to 48 hours.

### Brief Description of the Drawings

Fig. 1 shows the fouling reduction index in a solution of acryilonitrile treated according to the method of some embodiments of the present invention where oxygen was not removed from the pressure vessel.
Fig. 2 shows the fouling reduction index in a solution of acryilonitrile treated according to the method of some embodiments of the present invention where oxygen was removed from the pressure vessel.
Fig. 3 shows the fouling reduction index in a solution of acryilonitrile treated according to the method of some embodiments of the present invention where oxygen was not removed from the pressure vessel.
Fig. 4 shows the fouling reduction index in a solution of acryilonitrile treated according to the method of some embodiments of the present invention where oxygen was removed from the pressure vessel.

### Detailed Description

For clarity of disclosure, and not by way of limitation, the detailed description of the invention is divided into the following subsections that describe or illustrate certain features, embodiments or applications of the present invention.

The present invention provides a method, comprising: a. adding an effective amount of at least one compound selected from a substituted catechol of Formula 1 to a solution of a monomer selected from the group consisting of acrylonitrile, acrylic acid, vinylacetate, acrylates and acrolein; b. placing the monomer solution containing the at least one compound in a pressure vessel, pressurizing the pressure vessel and removing oxygen from the pressure vessel; and c. heating the monomer solution containing the at least one compound, wherein the at least one compound inhibits fouling by preventing polymerization of the monomer.

In some embodiments, the solution of acrylonitrile is formed via the ammoxidation of propylene. The method of ammoxidation is readily determined by one of ordinary skill in the art.

In some embodiments, the solution of acrolein is formed via the oxidation of propylene. The method of oxidation is readily determined by one of ordinary skill in the art.

In some embodiments, the solution of vinyl acetate is formed via the oxidation of ethylene. The method of oxindation is readily determined by one of ordinary skill in the art.

The term "mitigating fouling" or "preventing reducing fouling" or "inhibiting fouling" as used herein refers to the suppression or reduction of fouling, such as via the formation of polymer deposits.

In some embodiments, the effective amount of the at least one compound results in a 100% inhibition of fouling. In some embodiments, the effective amount of the at least one compound results in a 90% inhibition of fouling. In some embodiments, the effective amount of the at least one compound results in an 80% inhibition of fouling. In some embodiments, the effective amount of the at least one compound results in a 70% inhibition of fouling. In some embodiments, the effective amount of the at least one compound results in a 60% inhibition of fouling. In some embodiments, the effective amount of the at least one compound results in a 50% inhibition of fouling. In some embodiments, the effective amount of the at least one compound results in a 40% inhibition of fouling. In some embodiments, the effective amount of the at least one compound results in a 30% inhibition of fouling. In some embodiments, the effective amount of the at least one compound results in a 20% inhibition of fouling. In some embodiments, the effective amount of the at least one compound results in a 10% inhibition of fouling.

In some embodiments, the effective amount of the at least one compound results in a 10% reduction of fouling. In some embodiments, the effective amount of the at least one compound results in a 20% reduction of fouling. In some embodiments, the effective amount of the at least one compound results in a 30% reduction of fouling. In some embodiments, the effective amount of the at least one compound results in a 40% reduction of fouling. In some embodiments, the effective amount of the at least one compound results in a 50% reduction of fouling. In some embodiments, the effective amount of the at least one compound results in a 60% reduction of fouling. In some embodiments, the effective amount of the at least one compound results in a 70% reduction of fouling. In some embodiments, the effective amount of the at least one compound results in an 80% reduction of fouling. In some embodiments, the effective amount of the at least one compound results in a 90% reduction of fouling. In some embodiments, the effective amount of the at least one compound results in a 100% reduction of fouling.

In some embodiments, the at least one compound is a compound of Formula 1: 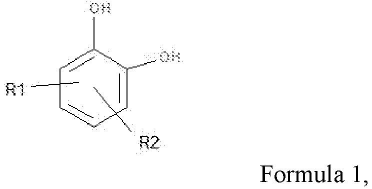 wherein R1 and R2 may be or include an alkyl group, an aryl group, an alkyl group, an alkyl group having a heteroatom, an aryl group having a heteroatom, and combinations thereof. The hetero atom may be or include, but is not limited to sulfur, nitrogen, oxygen, and combinations thereof. Non-limiting examples may be or include an ether group, a thiol group, and/or an ester group. R1 may be the same or different from R2.

In some embodiments, the compound of Formula 1 is selected from the group consisting of 4-*tert*-Butylcatechol, and 3,5-*tert*-Butylcatechol.

In some embodiments, the at least one compound is a compound of Formula 2: 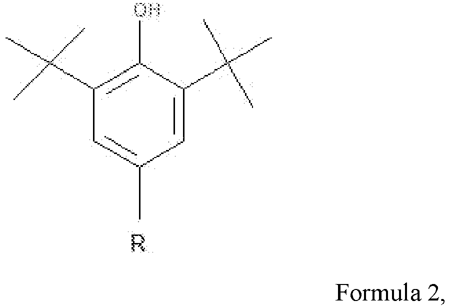 wherein R may be or include an alkyl group, an aryl group, an alkyl group having a heteroatom, an aryl group having a heteroatom, and combinations thereof. The heteroatom may be or include, but is not limited to sulfur, nitrogen, oxygen, and combinations thereof.

In some embodiments, the at least one compound is 4-hydroxy-2,2,6,6-tetramethylpiperidine-1-oxyl.

In some embodiments, the at least one compound is hydroxylamine.

In some embodiments, the at least one compound is phenylene diamine.

In some embodiments, the at least one compound is added to the monomer solution by direct injection to pump suction or by quill during the distillation, purification, and/or fractionation process. Alternatively, the at least one compound may be added to the equipment used for distillation, purification, and/or fractionation of the monomer. In one non-limiting embodiment, the additive may be injected into the feed, the reflux, and/or the boiler loop on a continuous basis, or the additive may be injected about every 0.5 hour to about 1 hour in an alternative embodiment.

In some embodiments, the effective amount of the at least one compound is 0.01 ppm independently to 10,000 ppm. In some embodiments the effective amount of the at least one compound is 1 ppm to 5000 ppm. In some embodiments the effective amount of the at least one compound is 1 ppm to 1200 ppm. In some embodiments the effective amount of the at least one compound is 1 ppm to 500 ppm. In some embodiments the effective amount of the at least one compound is 1 ppm to 100 ppm. In some embodiments the effective amount of the at least one compound is 1 ppm to 50 ppm. In some embodiments the effective amount of the at least one compound is 1 ppm to 40 ppm. In some embodiments the effective amount of the at least one compound is 1 ppm to 30 ppm. In some embodiments the effective amount of the at least one compound is 1 ppm to 20 ppm. In some embodiments the effective amount of the at least one compound is 1 ppm to 10 ppm. In some embodiments the effective amount of the at least one compound is 40 ppm.

In some embodiments, the at least one compound may be dispersed in a suitable aqueous carrier, such as water. The amount of aqueous carrier may have a ratio based on weight ranging from a 100:1 ratio to a 2:1 ratio, alternatively from a 20:1 ratio to a 2:1 ratio.

In some embodiments, two compounds are added to the monomer solution. The ratio of the first and second compound may be 1:1 to 1:10, or, alternatively, 1:1 to 1:5.

In some embodiments, the monomer solution containing the at least one compound is heated for up to 48 hours. In some embodiments, the monomer solution containing the at least one compound is heated for up to 24 hours. In some embodiments, the monomer solution containing the at least one compound is heated for up to 12 hours. In some embodiments, the monomer solution containing the at least one compound is heated for up to 6 hours. In some embodiments, the monomer solution containing the at least one compound is heated for up to 5 hours. In some embodiments, the monomer solution containing the at least one compound is heated for up to 4 hours. In some embodiments, the monomer solution containing the at least one compound is heated for up to 3 hours. In some embodiments, the monomer solution containing the at least one compound is heated for up to 2 hours. In some embodiments, the monomer solution containing the at least one compound is heated for up to 1 hour. In some embodiments, the monomer solution containing the at least one compound is heated for up to 0.5 hour.

In some embodiments, the monomer solution containing the at least one compound is heated up to 50 °C. In some embodiments, the monomer solution containing the at least one compound is heated up to 60 °C. In some embodiments, the monomer solution containing the at least one compound is heated up to 70 °C. In some embodiments, the monomer solution containing the at least one compound is heated up to 80 °C. In some embodiments, the monomer solution containing the at least one compound is heated up to 90 °C. In some embodiments, the monomer solution containing the at least one compound is heated up to 100 °C. In some embodiments, the monomer solution containing the at least one compound is heated up to 110 °C. In some embodiments, the monomer solution containing the at least one compound is heated up to 120 °C. In some embodiments, the monomer solution containing the at least one compound is heated up to 130 °C. In some embodiments, the monomer solution containing the at least one compound is heated up to 140 °C.

In some embodiments, the pressure within the pressure vessel is from -14 psi to 1400 psi. In some embodiments, the pressure within the pressure vessel is -14 psi. In some embodiments, the pressure within the pressure vessel is -10 psi. In some embodiments, the pressure within the pressure vessel is 10 psi. In some embodiments, the pressure within the pressure vessel is 20 psi. In some embodiments, the pressure within the pressure vessel is 50 psi. In some embodiments, the pressure within the pressure vessel is 100 psi. In some embodiments, the pressure within the pressure vessel is 200 psi. In some embodiments, the pressure within the pressure vessel is 200 psi. In some embodiments, the pressure within the pressure vessel is 400 psi. In some embodiments, the pressure within the pressure vessel is 500 psi. In some embodiments, the pressure within the pressure vessel is 600 psi. In some embodiments, the pressure within the pressure vessel is 700 psi. In some embodiments, the pressure within the pressure vessel is 800 psi. In some embodiments, the pressure within the pressure vessel is 900 psi. In some embodiments, the pressure within the pressure vessel is 1000 psi. In some embodiments, the pressure within the pressure vessel is 1100 psi. In some embodiments, the pressure within the pressure vessel is 1200 psi. In some embodiments, the pressure within the pressure vessel is 1300 psi. In some embodiments, the pressure within the pressure vessel is 1400 psi. The psi values given herein are to be converted into bar values of pressure using the conversion factor of 0.069 (1 psi= 0.069 bar).

In some embodiments, 100 % of the oxygen is removed from the pressure vessel. In some embodiments, 90 % of the oxygen is removed from the pressure vessel. In some embodiments, 80 % of the oxygen is removed from the pressure vessel. In some embodiments, 70 % of the oxygen is removed from the pressure vessel. In some embodiments, 60 % of the oxygen is removed from the pressure vessel. In some embodiments, 50 % of the oxygen is removed from the pressure vessel. In some embodiments, 40 % of the oxygen is removed from the pressure vessel. In some embodiments, 30 % of the oxygen is removed from the pressure vessel. In some embodiments, 20 % of the oxygen is removed from the pressure vessel. In some embodiments, 10 % of the oxygen is removed from the pressure vessel.

The present invention is further illustrated in the following examples.

### Examples

### Comparative Example 1: The Fouling Reduction Index Observed in a Solution of Acrylonitrile Treated According to the Method of Some Embodiments of the Present Invention Where Oxygen was not Removed from the Pressure Vessel

Four pressure vessels, containing 50 ml freshly distilled acrylonitrile (40 ppm AIBN mole concentration) with 20 ppm (weight) of either HQ, TBC, 3,5-TBC, or 2,6-di-tertbutyl-secondary phenol ('phenol"), was pressurized with air to 0.689 MPa (100 psi). A fresh acrylonitrile sample without any additive was also used as a control. All pressure vessels were heated at 90 °C for 4 hours. After the vessels were cooled down, the foulants were filtered, dried and weighed. The fouling reduction index was used to evaluate the effective of various additives. The fouling reduction index was calculated based on the following formula:

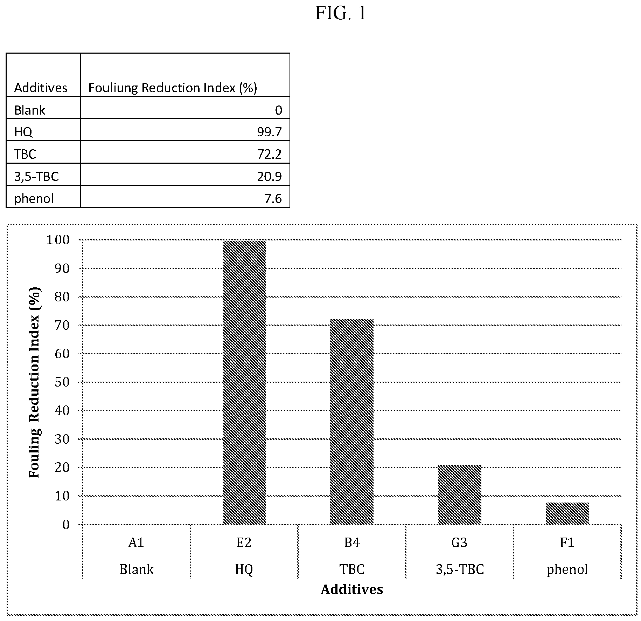

Fouling Reduction Index = 100 - (foulant formed without additives - foulant formed with additives) /foulant formed without additives x 100. The results are shown in Fig. 1.

HQ showed the best performance in the presence of the air. However TBC and 3,5-TBC can be used to reduce foulant formation. Due to the lower toxicity, TBC can be effectively used as HQ replacement to mitigate the fouling, which was caused by the free radical polymerizations.

### Example 2: The Fouling Reduction Index Observed in a Solution of Acrylonitrile Treated According to the Method of Some Embodiments of the Present Invention Where Oxygen was Removed from the Pressure Vessel

Four pressure vessels, containing 50 ml freshly distilled acrylonitrile (40 ppm AIBN mole concentration) with 10 ppm (weight) of either TBC, HTEMPO or hydroxylamine, was pressurized with nitrogen to 0.689 MPa (100 psi). A fresh acrylonitrile sample without any additive was also used as a control. All pressure vessels were heated at 90 °C for 4 hours.

After the vessels were cooled down, the foulants were filtered, dried and weighed. The fouling reduction index was used to evaluate the effective of various additives. The fouling reduction index was calculated based on the following formula:

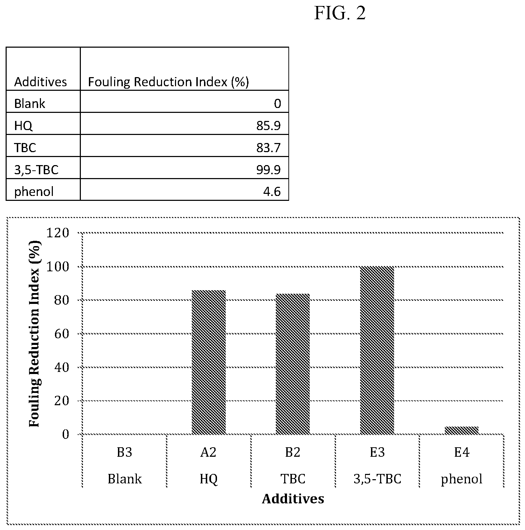

Fouling Reduction Index = 100- (foulant formed without additives - foulant formed with additives) /foulant formed without additives x 100. The results are shown in Fig. 2.

The test data indicated that removal of oxygen from the pressure vessel greatly enhanced the ability of TBC, 3,5-TBC, or 2,6-di-tertbutyl-secondary phenol ("phenol") to reduce fouling.

### Comparative Example 3: The Fouling Reduction Index Observed in a Solution of Acrylonitrile Treated According to the Method of Some Embodiments of the Present Invention Where Oxygen was not Removed from the Pressure Vessel

Three pressure vessels, containing 50 ml freshly distilled acrylonitrile (40 ppm AIBN mole concentration) with 10 ppm (weight) of either 10 ppm (weight) TBC and 10 ppm (weight) HTEMPO, or 10 ppm (weight) TBC plus 10 ppm (weight) DEHA, was pressurized with air to 0.689 MPa (100 psi). A fresh acrylonitrile sample without any additive was also used as a control. All pressure vessels were heated at 90 °C for 4 hours. After the vessels were cooled down, the foulants were filtered, dried and weighed. The fouling reduction index was used to evaluate the effective of various additives. The fouling reduction index was calculated based on the following formula:

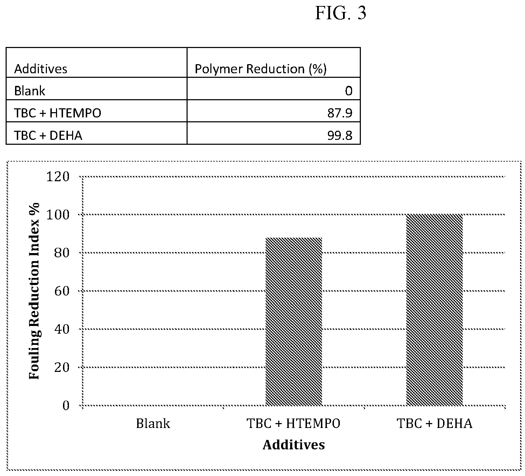

Fouling Reduction Index = 100 - (foulant formed without additives - foulant formed with additives) /foulant formed without additives x 100. The results are shown in Fig. 3.
The combination of TBC and DEHA showed the best performance in the presence of the air. The TBC/HTEMPO significantly reduced the formation of foulant. Therefore the synergetic effect of additives with TBC derivatives could significantly increase the performance of single components of phenol to mitigate the fouling.

### Example 4: The Fouling Reduction Index Observed in a Solution of Acrylonitrile Treated According to the Method of Some Embodiments of the Present Invention Where Oxygen was Removed from the Pressure Vessel

Three pressure vessels, containing 50 ml freshly distilled acrylonitrile (40 ppm AIBN mole concentration) with 10 ppm (weight) of either 10 ppm (weight) TBC and 10 ppm (weight) HTEMPO, or 10 ppm (weight) TBC plus 10 ppm (weight) DEHA, was pressurized with nitrogen to 0.689 MPa (100 psi). A fresh acrylonitrile sample without any additive was also used as a control. All pressure vessels were heated at 90 °C for 4 hours. After the vessels were cooled down, the foulants were filtered, dried and weighed. The fouling reduction index was used to evaluate the effective of various additives. The fouling reduction index was calculated based on the following formula:

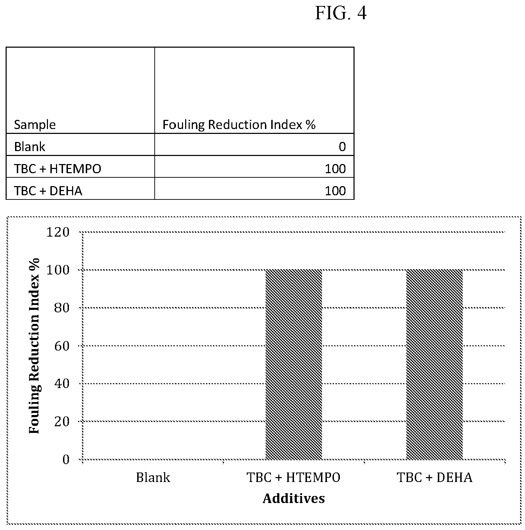

Fouling Reduction Index = 100 - (foulant formed without additives - foulant formed with additives) /foulant formed without additives x 100. The results are shown in Fig. 4.
The combination of TBC and DEHA showed the best performance in the presence of the air. The TBC/HTEMPO significantly reduced the formation of foulant. Therefore the synergetic effect of additives with TBC derivatives could significantly increase the performance of single components of phenol to mitigate the fouling.

The test data indicated that the synergistic effect of substituted catechol + HTEMPO and substituted catechol + DEHA could greatly reduce polymer formation. Therefore the combination of these additives can improve the reliability of alkene oxidation process by mitigating the fouling in the process.

## Claims

1. A method, comprising:
a. adding an effective amount of at least one compound selected from a substituted catechol of Formula 1: 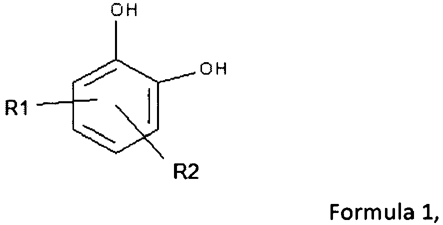 wherein R1 and R2 are independently selected from an alkyl group, an aryl group, an alkyl group having a heteroatom, an aryl group having a heteroatom, and combinations thereof to a solution of a monomer selected from the group consisting of acrylonitrile, acrylic acid, vinylacetate, acrylates and acrolein;
b. placing the monomer solution containing the at least one compound in a pressure vessel, pressurizing the pressure vessel and removing oxygen from the pressure vessel; wherein the pressure within the pressure vessel is from -0.0965 MPa (-14 psi) to 9.652 MPa (1400 psi) and
c. heating the monomer solution containing the at least one compound to a temperature from 50 °C to 140 °C,
wherein the at least one compound inhibits fouling by preventing polymerization of the monomer.

2. The method of claim 1, wherein the substituted catechol is selected from the group consisting of 4-*tert*-Butylcatechol, and 3,5-*tert*-Butylcatechol.

3. The method of claim 1, wherein the monomer solution containing the at least one compound is heated for a period of time from 0.5 to 48 hours.

## Patentansprüche

1. Verfahren, umfassend:
a. Hinzufügen einer wirksamen Menge mindestens einer Verbindung, ausgewählt aus einem substituierten Catechol der Formel 1: 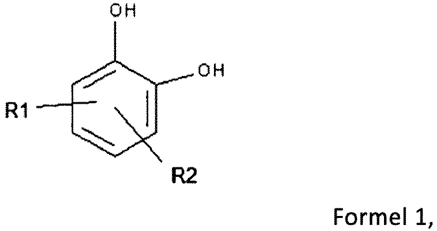 worin R1 und R2 unabhängig voneinander ausgewählt sind aus einer Alkylgruppe, einer Arylgruppe, einer Alkylgruppe mit einem Heteroatom, einer Arylgruppe mit einem Heteroatom und Kombinationen davon, zu einer Lösung von einem Monomer ausgewählt aus der Gruppe bestehend aus Acrylnitril, Acrylsäure, Vinylacetat, Acrylaten und Acrolein;
b. Anordnen der die mindestens eine Verbindung enthaltenden Monomerlösung in einem Druckgefäß, unter Druck setzen des Druckbehälters und Entfernen von Sauerstoff aus dem Druckgefäß; wobei der Druck innerhalb des Druckbehälters von -0,0965 MPa (-14 psi) bis 9,652 MPa (1400 psi) beträgt, und
c. Erhitzen der Monomerlösung, die die mindestens eine Verbindung enthält, auf eine Temperatur von 50 °C bis 140 °C,
wobei die mindestens eine Verbindung den Bewuchs durch Verhindern der Polymerisation des Monomers hemmt.

2. Verfahren nach Anspruch 1, wobei das substituierte Catechol ausgewählt ist aus der Gruppe bestehend aus 4-*tert*-Butylcatechol und 3,5-*tert*-Butylcatechol.

3. Verfahren nach Anspruch 1, wobei die Monomerlösung, die die mindestens eine Verbindung enthält, für einen Zeitraum von 0,5 bis 48 Stunden erhitzt wird.

## Revendications

1. Procédé comprenant :
a. l'ajout d'une quantité efficace d'au moins un composé choisi parmi un catéchol substitué de Formule 1 : 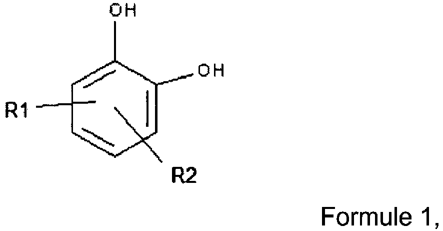 dans lequel R1 et R2 sont indépendamment choisis parmi un groupe alkyle, un groupe aryle, un groupe alkyle ayant un hétéroatome, un groupe aryle ayant un hétéroatome, et des combinaisons de ceux-ci à une solution d'un monomère choisi dans le groupe constitué d'acrylonitrile, acide acrylique, vinylacétate, acrylates et acroléine ;
b. la mise en place de la solution de monomère contenant l'au moins un composé dans un récipient à pression, la mise sous pression du récipient à pression et l'élimination de l'oxygène hors du récipient à pression ; dans lequel la pression à l'intérieur du récipient à pression va de -0,0965 MPa (-14 psi) à 9,652 MPa (1400 psi) et
c. le chauffage de la solution de monomère contenant l'au moins un composé à une température allant de 50 °C à 140 °C,
dans lequel l'au moins un composé inhibe l'encrassement en empêchant la polymérisation du monomère.

2. Procédé selon la revendication 1, dans lequel le catéchol substitué est choisi dans le groupe constitué de 4-*tert*-butylcatéchol, et 3,5-*tert*-butylcatéchol.

3. Procédé selon la revendication 1, dans lequel la solution de monomère contenant l'au moins un composé est chauffée pendant un laps de temps allant de 0,5 à 48 heures.
